(19) Europäisches Patentamt
European Patent Office
Office européen des brevets

(11) Veröffentlichungsnummer: **0 166 214 B1**

(12) # EUROPÄISCHE PATENTSCHRIFT

(45) Veröffentlichungstag der Patentschrift: **26.07.89**

(51) Int. Cl.[4]: **C 07 C 69/95**, C 07 C 67/313

(21) Anmeldenummer: **85106358.6**

(22) Anmeldetag: **24.05.85**

(54) **Verfahren zur Herstellung von Succinylobernsteinsäuredialkylestern.**

(30) Priorität: **26.06.84 DE 3423548**

(43) Veröffentlichungstag der Anmeldung: **02.01.86 Patentblatt 86/1**

(45) Bekanntmachung des Hinweises auf die Patenterteilung: **26.07.89 Patentblatt 89/30**

(84) Benannte Vertragsstaaten: **AT CH DE FR GB IT LI**

(56) Entgegenhaltungen:
**EP-A- 0 057 873**

**PATENT ABSTRACTS OF JAPAN, Seite 2166 C 77; & JP - A 52 59135 (SHOWA DENKO) 16.05.1977**

(73) Patentinhaber: **Chemie Linz Gesellschaft m.b.H., St.Peter-Strasse 25, A-4021 Linz (AT)**
(84) Benannte Vertragsstaaten: **CH FR GB IT LI AT**

(73) Patentinhaber: **Lentia Gesellschaft mit beschränkter Haftung, Arabellastrasse 4 Postfach 81 05 08, D-8000 München 81 (DE)**
(84) Benannte Vertragsstaaten: **DE**

(72) Erfinder: **Schneider, Rudolf, Dipl. Ing. Dr., Hauserstrasse 12, A-4040 Linz (AT)**
Erfinder: **Kahofer, Leo, Dr., Verstorben (AT)**

(74) Vertreter: **Kunz, Ekkehard, Dr., Chemie Holding AG Patentwesen St. Peter-Strasse 25, A-4021 Linz (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft die Herstellung von Succinylobernsteinsäuredialkylestern durch Kondensation von Bernsteinsäuredialkylester in Gegenwart von Alkalialkoholaten.

Succinylobernsteinsäuredialkylester sind Zwischenprodukte für die Herstellung von Chinacridonfarbstoffen, die vor allem als lichtechte Pigmente für Autolacke Anwendung finden. Succinylobernsteinsäuredialkylester werden in der Praxis entsprechend der Gleichung:

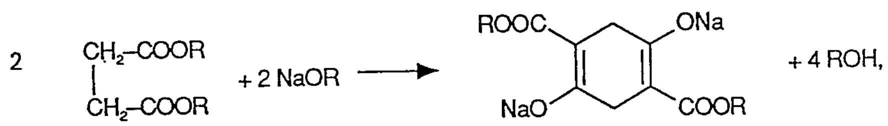

in der R für ein niederes Alkyl steht, durch Umsetzung von Bernsteinsäuredialkylestern mit Alkalialkoholat in einem Lösungsmittel hergestellt, wobei zur Erreichung guter Ausbeuten ein Überschuss an Alkalialkoholat nötig ist. In erster Stufe fällt das Alkalisalz des Succinylobernsteinsäureesters aus, welches dann durch Ansäuern in den freien Ester überführt und abfiltriert werden kann.

In der EPA-57 873 wird die Herstellung von Succinylobernsteinsäuredimethylester in Methanol als Lösungsmittel beschrieben, wobei man Bernsteinsäuredimethylester bei 80 bis 120°C zu einem 20- bis 80%igen Überschuss einer 35- bis 45-Gew.%igen methanolischen Natriummethylatlösung zusetzt, das Methanol sodann teilweise abdestilliert und anschliessend 6 bis 12 Stunden am Rückfluss kocht. Die Ausbeute beträgt 76%, bezogen auf den eingesetzten Bernsteinsäuredimethylester. Die Verwendung von N-Methylpyrrolidon bzw. Dimethylformamid als Lösungsmittel wird in der DE-PS 1 082 907 vorgeschlagen. Dabei werden Bernsteinsäurediäthylester und überschüssiges Natriummethylat in dem jeweiligen Lösungsmittel durch 1- bis 4stündiges Erhitzen auf Temperaturen bis zu 150°C umgesetzt und sodann noch bis zu 10 Stunden bei 90 bis 120°C weiter erhitzt.

In den US-PS 2 821 541 und US-PS 3 024 268 wird die Umsetzung von niederen Bernsteinsäuredialkylestern mit einem Überschuss an Natriumalkoholat, das unmittelbar vorher in der Reaktionsapparatur unter Stickstoffatmosphäre aus metallischem Natrium und Alkohol hergestellt wurde, in Dowtherm® als Lösungsmittel bei 90 bis 120°C beschrieben. Die benötigte Gesamtzeit für die Reaktion beträgt annähernd 20 Stunden, wobei Ausbeuten bis zu 80% erzielt werden.

In der JA-Kokai 75-19 738 wird Bernsteinsäurediäthylester zu einer Lösung von Natriumäthylat in Xylol zugesetzt und nach zweistündiger Reaktion bei 110°C Succinylobernsteinsäurediäthylester mit einer Ausbeute von 74% erhalten. Xylol in Mischung mit Dimethylsulfoxid wird in der JA-69-27 577 und in der JA-Kokai 77-59 135 als Lösungsmittel vorgeschlagen. Je Mol Bernsteinsäureester wurden 0,8 bis 3 Mol Alkalialkoholat verwendet. Da die Ausbeuten bei einem Molverhältnis von 0,8 jedoch bei nur 64% lagen, wurde zur Erzielung von verbesserten Ausbeuten von bis zu 80% ein Überschuss an Alkalialkoholat eingesetzt. Ebenso sank die Ausbeute auf 55% bei alleiniger Verwendung von Xylol als Lösungsmittel ohne Zusatz von Dimethylsulfoxid.

Alle diese bekannten Verfahren besitzen den gemeinsamen Nachteil, dass die Umsetzung in einem Fremdlösungsmittel durchgeführt wird, welches anschliessend an die Reaktion wieder abgetrennt, gereinigt und entwässert werden muss bzw. teilweise verlorengeht. Besonders aufwendig ist die Aufarbeitung von mit Wasser mischbaren Lösungsmitteln wie z.B. Alkoholen, Dimethylsulfoxid, Dimethylformamid oder N-Methylpyrrolidon bzw. in den Fällen, wo zur Erzielung besserer Ausbeuten Lösungsmittelgemische vorliegen. Die Verfahren, die metallisches Natrium verwenden, erfordern wegen der gefährlichen Arbeitsweise besondere Sicherheitsvorkehrungen und sind ausserdem sehr zeitaufwendig. Ein weiterer entscheidender Nachteil der bekannten Verfahren liegt auch darin, dass der verwendete Überschuss an Natriumalkoholat im Zuge der Aufarbeitung des Reaktionsgemisches hydrolisiert und damit verlorengeht, wobei sich dadurch neben dem daraus resultierenden höheren Materialverbrauch auch eine zusätzliche Belastung der Abwässer ergibt.

Es wurde nun überraschenderweise gefunden, dass im Gegensatz dazu die angeführten Nachteile der bekannten Verfahren dadurch ausgeschaltet werden, dass man den als Reaktionskomponente eingesetzten, im Reaktionsgefäss vorgelegten Bernsteinsäuredialkylester gleichzeitig auch als Lösungs- oder Verdünnungsmittel für die Umsetzung verwendet.

Gegenstand der Erfindung ist demnach ein Verfahren zur Herstellung von Succinylobernsteinsäuredialkylestern, bzw. deren Alkalisalzen durch Kondensation von Bernsteinsäuredialkylestern in Gegenwart von Alkalialkoholaten, wobei das Alkalialkoholat in Form einer 5- bis 45%igen alkoholischen Lösung eingesetzt wird, bei Temperaturen von über 45°C, mindestens aber bei der Siedetemperatur des verwendeten Alkohols unter den gewählten Reaktionsbedingungen, das dadurch gekennzeichnet ist, dass man das Alkalialkoholat zu dem vorgelegten Bernsteinsäuredialkylester bei Normaldruck oder vermindertem Druck in einer Menge zusetzt, dass zur Reaktion pro Mol Alkalialkoholat mindestens 1,5 Mol Bernsteinsäuredialkylester verwendet werden und dabei gleichzeitig den Alkohol abdestilliert, worauf man in sich bekannter Weise die Reaktionsmischung ansäuert und den abgeschiedenen Succinylobernsteinsäuredialkylester abtrennt.

Ein wesentlicher Vorteil des erfindungsgemässen Verfahrens besteht demnach darin, dass für die Reaktion Bernsteinsäuredialkylester sowohl als eine der Reaktionskomponenten als auch als Lösungsmittel verwendet wird. Dadurch entfallen die Operationen zur Rückgewinnung bzw. der Lösungsmittelgemische, wie sie bei Verwendung eines Fremdlösungsmittels erforderlich sind. Der nach erfolgter Reaktion verbleibende Überschuss an Bernsteinsäuredialkylester wird vorteilhafterweise einem neuen Reaktionsansatz zur Herstellung von Succinylobernsteinsäuredialkylester zugesetzt. Die Aufarbeitung erfolgt entweder durch Destillation, in den meisten Fällen genügt dabei jedoch eine Trocknung durch Abdestillieren eines wässrigen Vorlaufs. Für den durch die alkoholische Alkalialkoholatlösung in die Reaktionsmischung gelangten Alkohol ist keine spezielle Aufarbeitung erforderlich, da er sofort im Verlaufe der Reaktion, gemeinsam mit dem durch die Umsetzung entstehenden Alkohol, abdestilliert wird. Dazu ist es erforderlich, das Reaktionsgemisch mindestens auf die Siedetemperatur des entsprechenden Alkohols aufzuheizen. Durch die laufende Entfernung sowohl des zugesetzten als auch des entstehenden Alkohols aus dem Reaktionsgleichgewicht durch Abdestillieren während der Reaktion, wird ein sehr hoher Umsetzungsgrad erreicht. Besonders leicht gelingt das Abdestillieren des Alkohols, wenn während der Reaktion im Vakuum gearbeitet wird. Dadurch ist es auch möglich, die Reaktionstemperatur zu erniedrigen.

Die Ausbeuten von bis zu 86% bezogen auf das eingesetzte Alkalialkoholat liegen im Vergleich zu ca. 50% bei den bekannten Verfahren, bei denen das überschüssige Alkalialkoholat im Verlaufe der Aufarbeitung des Reaktionsgemisches hydrolisiert und anschliessend verworfen werden muss, sehr hoch. Bezogen auf den Bernsteinsäuredialkylester, der beim vorliegenden erfindungsgemässen Verfahren im Überschuss eingesetzt wird, welcher jedoch wieder rückgewonnen wird, liegen die Ausbeuten mit bis zu 76% etwa im Bereich der in der Literatur angeführten Werte.

Ein weiterer wesentlicher Vorteil des kontinuierlichen Abdestillierens des Alkohols aus dem Reaktionsgemisch ergibt sich aus der Tatsache, dass die Reaktion nach erfolgter Zugabe des Alkoholats, was im Durchschnitt etwa eine Stunde dauert, beendet ist. Im Gegensatz dazu ist es bei den bekannten Verfahren notwendig, im Anschluss an die Zugabe des Bernsteinsäureesters je nach Verfahren für weitere 2 bis 12 Stunden am Rückfluss zu kochen. Durch die damit erzielbare, vergleichsweise kurze Reaktionszeit zeichnet sich das erfindungsgemässe Verfahren zusätzlich zu dem bereits erwähnten Wegfall eines speziellen Lösungsmittels und dem geringeren Rohstoffverbrauch auch in bezug auf Energieverbrauch und Arbeitszeit durch eine verbesserte Wirtschaftlichkeit aus.

Die Mindesttemperatur während der Reaktion ist vom jeweils angewandten Druck abhängig und muss über der Siedetemperatur des jeweils vorliegenden Lösungsmittelgemisches aus Bernsteinsäuredialkylester und Alkohol liegen, bei Normaldruck und Verwendung von Natriummethylat also über 65°C, im Vakuum über 45°C. Um jedoch den Alkohol möglichst vollständig abdestillieren zu können und somit vom Beginn weg möglichst wenig Alkohol im Gleichgewicht vorliegen zu haben, hat es sich als vorteilhaft erwiesen, eine Temperatur von 80–150°C, vorzugsweise von 100–130°C einzuhalten.

Die Entfernung des Alkohols aus dem Reaktionsgemisch gelingt besonders effektiv, wenn die Reaktion im Vakuum durchgeführt wird, wobei der Alkohol besonders leicht abdestilliert. Dabei ist jeder Druck, der unter dem Atmosphärendruck liegt, vorteilhaft, wobei als zweckmässige Untergrenze das mit der Wasserstrahlpumpe erreichbare Vakuum von ca. 20 mbar gesetzt wird. Bei Verwendung anderer Vakuumpumpen ist jedoch auch ein Arbeiten bei Drucken unter 20 mbar möglich. Besonders bevorzugt wird bei einem Vakuum von 100 bis 450 mbar gearbeitet.

Die Durchführung der Reaktion gestaltet sich besonders einfach, wenn das Alkoholat in Form einer 20- bis 30%igen alkoholischen Alkalialkoholatlösung verwendet wird.

Mit dem erfindungsgemässen Verfahren sind alle jene Succinylobernsteinsäurealkylester vorteilhaft herstellbar, deren Alkoholkomponenten durch Destillation, zweckmässig bei Temperaturen bis zu etwa 200°C abdestillierbar sind. Sehr gut anwendbar ist es für Ester mit Alkoholen bis zu 6 C-Atomen, vorteilhaft für Ester mit Alkoholen von 1 bis 4 C-Atomen. Unter den mit dem erfindungsgemässen Verfahren herstellbaren Verbindungen sind Succinylobernsteinsäuredimethylester und Succinylobernsteinsäurediäthylester besonders bevorzugt, da sie gefragte Zwischenprodukte in der Chinacridondarstellung sind. Ihre Darstellung erfolgt entsprechend dem erfindungsgemässen Verfahren z.B. durch Umsetzung von Bernsteinsäuredimethylester mit einer Lösung von Natriummethylat in Methanol, bzw. durch Umsetzung von Bernsteinsäurediäthlyester mit einer Lösung von Natriumäthylat in Äthanol.

Da der Bernsteinsäuredialkylester sowohl als Reaktionskomponente als auch an Stelle eines Fremdlösungsmittels eingesetzt wird, muss er im Überschuss, bezogen auf das eingesetzte Alkalialkoholat, vorliegen. Die Menge des Bernsteinsäuredialkylesters muss mindestens so gross sein, dass sich die bei der Reaktion bildende Suspension noch rühren lässt, was bis zu einem Molverhältnis Bernsteinsäuredialkylester zu Alkalialkoholat von 1,5:1 der Fall ist. Um eine besonders günstige Rührbarkeit zu gewährleisten, hat sich ein Molverhältnis von 2:1 bis 10:1 als vorteilhaft erwiesen, wobei ein Molverhältnis von 4:1 bis 5:1 bevorzugt wird.

Der freie Succinylobernsteinsäuredialkylester wird durch Ansäuern des gelösten Alkalisalzes gebildet, wobei jede organische oder anorganische Säure, einschliesslich Kohlendioxid sowie saure Salze, verwendet werden können. Eine bevorzugte Möglichkeit stellt z. B. die Verwendung einer 10- bis 20%igen wässrigen Schwefelsäure dar.

Das Verfahren gestaltet sich dadurch besonders wirtschaftlich, dass man die nach der Abtrennung des Succinylobernsteinsäuredialkylesters anfallende Mutterlauge, die aus einer wässrigen Phase und aus einer organischen Phase mit nicht umgesetztem Bernsteinsäuredialkylester besteht, nach Phasentrennung wieder zur Herstellung von Succinylobernsteinsäuredialkylester einsetzt, wobei die wässrige Phase verworfen wird und der Bernsteinsäuredialkylester wieder für einen neuen Ansatz verwendet wird. Dabei kann der Bernsteinsäuredialkylester durch Destillation gereinigt werden, in den meisten Fällen genügt es jedoch, ihn nur durch Abdestillieren eines wässrigen Vorlaufs zu trocknen. Dieser wässrige Vorlauf kann gemeinsam mit dem Waschwasser, welches zum Waschen des abgetrennten Succinylobernsteinsäuredialkylesters gebraucht wurde, vorteilhafterweise zur Bereitung der zum Ansäuern benötigten Säure verwendet werden, wodurch auch der darin gelöste Bernsteinsäuredialkyester wieder in den Prozess rückgeführt wird.

Die im folgenden angeführten Beispiele sollen zur näheren Erläuterung des erfindungsgemässen Verfahrens dienen:

Beispiel 1

In einem 1-l-Vierhalsrundkolben mit Rührer, Thermometer, Tropftrichter, Vigreuxkolonne mit Destillationseinrichtung und Vakuumpumpe werden 657 g (4.5 Mol) trockener Bernsteinsäuredimethylester vorgelegt und bei einem Vakuum von 450 mbar auf 105°C erwärmt. Dann werden 180 g 30%ige Natriummethylat-Lösung (1 Mol) in Methanol innerhalb einer Stunde zugetropft und gleichzeitig Methanol über die Kolonne abdestilliert, so dass die Temperatur bei 105°C bleibt. Danach wird die Suspension gekühlt, in 327 g 15%ige Schwefelsäure eingerührt, das Dimethylsuccinylosuccinat abfiltriert, mit 200 ml Wasser gewaschen und getrocknet. Es werden 96 g Dimethylsuccinylosuccinat, Schmelzpunkt 154–155°C, erhalten, (84,3% Ausbeute bezogen auf eingesetztes Na-Methylat). Das zweiphasige Filtrat wird getrennt, die organische Phase wird im Vakuum destilliert. Der wasserhaltige Destillationsvorlauf wird getrennt aufgefangen. Es werden insgesamt 435 g Bernsteinsäuredimethylester zurückgewonnen. Ausbeute bezogen auf Ester: 65,8%.

Beispiel 2

Es wird wie im Beispiel 1 verfahren, jedoch werden 350 g des im Beispiel 1 destillierten Bernsteinsäuredimethylesters und 307 g frischer Ester eingesetzt. Das Waschwasser von Beispiel 1 sowie das Wasser des Destillationsvorlaufes wird zur Bereitung der 15%igen Schwefelsäure benützt. Es werden 98 g Dimethylsuccinylosuccinat, Schmelzpunkt 154–155°C, erhalten (85,9% Ausbeute bezogen auf Na-Methylat).

Bei der Destillation der organischen Phase des Filtrates werden insgesamt 461 g Bernsteinsäuredimethylester zurückgewonen. Ausbeute bezogen auf Ester: 74,5%.

Beispiel 3

Der Versuch wird analog zu Beispiel 1, jedoch ohne Vakuum ausgeführt. Dabei werden 95 g Dimethylsuccinylosuccinat, Schmelzpunkt 154–155°C, erhalten. Die organische Phase des Filtrates, der rohe Bernsteinsäuredimethylester wird nicht zur Gänze destilliert, sondern durch Abdestillieren eines Vorlaufes getrocknet. Es werden 361 g gelb gefärbter, trockener Rohester erhalten.

Beispiel 4

Der Versuch wird analog zu Beispiel 2, jedoch ohne Vakuum durchgeführt. Es werden jedoch anstelle von 350 g destilliertem Ester 361 g getrockneter Rohester von Beispiel 3 eingesetzt. Dabei werden 90 g Dimethylsuccinylosuccinat, Schmelzpunkt 153–155°C, erhalten.

Beispiel 5

In einer Apparatur analog Beispiel 1 werden 784 g Bernsteinsäurediäthylester vorgelegt und auf 110°C erwärmt. Dann werden bei einem Vakuum von 450 mbar 340 g 20%ige Na-Äthylat-Lösung, entsprechend einem molaren Verhältnis von 4,5 : 1, zugetropft, und gleichzeitig wird Äthanol über die Kolonne abdestilliert, so dass die Temperatur bei 110°C bleibt. Nach Ende des Zutropfens wird die Suspension gekühlt, in 490 g 10%ige Schwefelsäure eingerührt, filtriert, mit 300 ml Wasser gewaschen und getrocknet. Es werden 106 g Diäthylsuccinylosuccinat, Schmelzpunkt 126–128°C, erhalten (82,7% Ausbeute bezogen auf Na-Äthylat).

Das zweiphasige Filtrat wird getrennt, die organische Phase wird im Vakuum destilliert. Inklusive Vorlauf werden 555 g Bernsteinsäurediäthylester zurückgewonnnen (Ausbeute bezogen auf Ester: 76,1%).

Beispiel 6

In einer Apparatur analog zu Beispiel 1 wurden 657 g Bernsteinsäuredimethylester auf 45°C erwärmt und volles Wasserstrahlvakuum angelegt. Sodann wurden innerhalb von 1 Stunde 180 g 30%iges Na-Methylat in Methanol zugetropft, gleichzeitig verdampfte das Methanol, welches jedoch nicht kondensiert werden konnte. Anschliessend lässt man 2 Stunden nachreagieren. Der gesamte Ansatz wird sodann in 330 g 15%ige Schwefelsäure gegossen, kurze Zeit gerührt, der Succinylobernsteinsäuredimethylester abfiltriert, mit Wasser gewaschen und getrocknet.

Der erhaltene Succinylobernsteinsäuredimethylester hatte einen Schmelzpunkt von 154–155 °C.

Beispiel 7

Analog zu Beispiel 1 wurden 1316 g Dimethylsuccinat (9 Mol) vorgelegt, auf 80 °C erwärmt und ein Vakuum von 450 mbar angelegt. Anschliessend wurden 240 g einer 45%igen, auf 50 °C erwärmten Na-Methylat-Lösung (2 Mol) zugetropft und gleichzeitig Methanol abdestilliert. Danach erhöhte man das Vakuum auf 100 mbar und liess 30 Minuten nachreagieren. Die Reaktionslösung wurde sodann in 900 g 10%ige Schwefelsäure gegossen, filtriert, mit Wasser gewaschen und bei 60 °C getrocknet. Es wurden 193 g Succinylobernsteinsäuredimethylester, entsprechend einer Ausbeute von 84,6% bezogen auf Na-Methylat, erhalten.

Beispiel 8

In einer Versuchsanordnung analog zu Beispiel 1, jedoch bei Normaldruck, wurden 5 Mol Bernsteinsäuredimethylester auf 170 °C erwärmt und während 1 Stunde 0,5 Mol einer 5%igen methanolischen Na-Methylatlösung zugetropft und das Methanol abdestilliert.

Anschliessend wurde das Reaktionsgemisch abgekühlt und in 300 g 10%ige Essigsäure gegossen, sodann filtriert, gewaschen und getrocknet. Es wurden 19 g Succinylobernsteinsäuredimethylester, entsprechend einer Ausbeute von 33%, erhalten.

Beispiel 9

Analog zu Beispiel 1 wurden 2 Mol Bernsteinsäuredimethylester auf 110 °C erwärmt und anschliessend innerhalb einer halben Stunde bei einem Vakuum von 450 mbar 1 Mol einer 30%igen Na-Methylatlösung zugetropft, wobei gleichzeitig Methanol abdestillierte. Anschliessend wurde abgekühlt, mit 15%iger Schwefelsäure angesäuert, filtriert, mit Wasser gewaschen und getrocknet. Es wurden 72,9 g Succinylobernsteinsäuredimethylester, entsprechend einer Ausbeute von 64%, erhalten.

## Patentansprüche

1. Verfahren zur Herstellung von Succinylobernsteinsäuredialkylestern, bzw. deren Alkalisalzen durch Kondensation von Bernsteinsäuredialkylestern in Gegenwart von Alkalialkoholaten, wobei das Alkalialkoholat in Form einer 5- bis 45%igen alkoholischen Lösung eingesetzt wird, bei Temperaturen von über 45 °C, mindestens aber bei der Siedetemperatur des verwendeten Alkohols unter den gewählten Reaktionsbedingungen, dadurch gekennzeichnet, dass man das Alkalialkoholat zu dem vorgelegten Bernsteinsäuredialkylester bei Normaldruck oder vermindertem Druck in einer Menge zusetzt, dass zur Reaktion pro Mol Alkalialkoholat mindestens 1,5 Mol Bernsteinsäuredialkylester verwendet werden und dabei gleichzeitig den Alkohol abdestilliert, worauf man in an sich bekannter Weise die Reaktionsmischung ansäuert und den abgeschiedenen Succinylobernsteinsäuredialkylester abtrennt.

2. Verfahren nach Anspruch 1, dadurch gekennzeichnet, dass die Reaktion bei einem Druck von 100 bis 450 mbar durchgeführt wird.

3. Verfahren nach den Ansprüchen 1 und 2, dadurch gekennzeichnet, dass eine 10- bis 30%ige Alkalialkoholatlösung verwendet wird.

4. Verfahren nach den Ansprüchen 1 bis 3, dadurch gekennzeichnet, dass eine Lösung von Natriummethylat in Methanol zu Bernsteinsäuredimethylester zugesetzt wird.

5. Verfahren nach den Ansprüchen 1 bis 3, dadurch gekennzeichnet, dass eine Lösung von Natriumäthylat in Äthanol zu Bernsteinsäurediäthylester zugesetzt wird.

6. Verfahren nach den Ansprüchen 1 bis 5, dadurch gekennzeichnet, dass das Molverhältnis Bernsteinsäuredialkylester zu Alkalialkoholat 2:1 bis 10:1 beträgt.

7. Verfahren nach Anspruch 6, dadurch gekennzeichnet, dass das Molverhältnis Bernsteinsäuredialkylester zu Alkalialkoholat 4:1 bis 5:1 beträgt.

8. Verfahren nach den Ansprüchen 1 bis 7, dadurch gekennzeichnet, dass man den nach der Abtrennung des Succinylobernsteinsäuredialkylesters in der organischen Phase der anfallenden Mutterlauge enthaltenen, nicht umgesetzten Bernsteinsäuredialkylester nach Phasentrennung wieder als Ausgangsmaterial zur Herstellung von Succinylobernsteinsäuredialkylester einsetzt.

## Claims

1. Process for preparing dialkyl succinylsuccinates, or alkali metal salts thereof, by condensation of dialkyl succinates in the presence of alkali metal alcoholates, the alkali metal alcoholate being used in the form of a 5 to 45% strength alcoholic solution, at temperatures of above 45 °C, but at least at the boiling point, under the chosen reaction conditions, of the alcohol used, characterized in that the alkali metal alcoholate is added to the initially introduced dialkyl succinate under atmospheric or reduced pressure in such an amount that for the reaction at least 1.5 moles of dialkyl succinate are used per mole of alkali metal alcoholate, and at the same time the alcohol is distilled off, whereafter the reaction mixture is acidified in a conventional manner and the precipitated dialkyl succinylsuccinate is separated off.

2. Process according to Claim 1, charaterized in that the reaction is carried out under a pressure of 100 to 450 mbar.

3. Process according to Claims 1 and 2, characterized in that a 20 to 30% strength alkali alcoholate solution is used.

4. Process according to Claims 1 to 3, characterized in that a solution of sodium methylate in methanol is added to dimethyl succinate.

5. Process according to Claims 1 to 3, characterized in that a solution of sodium ethylate in ethanol is added to diethyl succinate.

6. Process according to Claims 1 to 5, characterized in that the molar ratio of the alkyl succinate: alkali metal alcoholate is 2:1 to 10:1.

7. Process according to Claim 6, characterized in that the molar ratio of dialkyl succinate: alkali metal alcoholate is 4:1 to 5:1.

8. Process according to Claims 1 to 7, characterized in that the unconverted dialkyl succinate present in the organic phase of the mother liquor obtained on removal of the dialkyl succinylsuccinate is reused after phase separation as a starting material for preparing dialkyl succinylsuccinate.

## Revendications

1. Procédé de préparation de succinylsuccinates de dialkyle, ou de leurs sels alcalins, par condensation de succinate de dialkyle en présence d'alcoolates alcalins, en utilisant l'alcoolate alcalins sous la forme d'une solution alcoolique à 5 à 45%, à des températures supérieures à 45°C, mais au moins à la température d'ébullition de l'alcool utilisé dans les conditions de réaction choisies, caracterisé en ce qu'on ajoute l'alcoolate alcalin au succinate de dialkyle présent à la pression normale ou sous pression réduite dans une proportion telle qu'on utilise pour la réaction, par mole d'alcoolate alcalin, au moins 1,5 mole de succinate de dialkyle et l'on élimine alors en meme temps l'alcool par destillation, puis l'on acidifie le mélange réactionnel de façon connue en soi et l'on sépare le succinylsuccinate de dialkyle déposé.

2. Procédé suivant la revendication 1, caractérisé en ce qu'on effectue la réaction à une pression de 100 à 450 millibars.

3. Procédé suivant les revendications 1 et 2, caractérisé en ce qu'on utilise une solution d'alcoolate alcalin à 20 à 30%.

4. Procédé suivant les revendications 1 à 3, caractérisé en ce qu'on ajoute à du succinate de diméthyle une solution de méthylate de sodium dans le méthanol.

5. Procédé suivant les revendications 1 à 3, caractérisé en ce qu'on ajoute à du succinate de diéthyle une solution d'éthylate de sodium dans l'éthanol.

6. Procédé suivant les revendications 1 à 5, caractérisé en ce que la proportion molaire sucinate de dialkyle: alcoolate alcalin est de 2:1 à 10:1.

7. Procédé suivant la revendication 6, caractérisé en ce que la proportion molaire succinate de dialkyle: alcoolate alcalin est de 4:1 à 5:1.

8. Procédé suivant les revendications 1 à 7, caractérisé en ce qu'on réutilise le succinate de dialkyle n'ayant pas réagi, contenu dans la phase organique de la lessive-mère apparaissant après la séparation du succinylsuccinate de dialkyl, après la séparation des phases, comme matière de départ pour la préparation de succinylsuccinate de dialkyle.